# EUROPEAN PATENT APPLICATION

(11) **EP 4 215 241 A1**
(43) Date of publication of application: **26.07.2023**
(21) Application number: 21869440.4
(22) Date of filing: 16.09.2021
(51) Int. Cl.: A61N 5/06, A61F 9/007

(54) **DEVICE AND METHOD FOR INHIBITING CHOROIDAL THINNING**

(30) Priority: 16.09.2020 JP 2020155389
(71) Applicant: Tsubota Laboratory, Inc., Tokyo 160-0016 (JP)
(72) Inventor: JIANG Xiaoyan, Tokyo 160-0016 (JP); MORI Kiwako, Tokyo 160-0016 (JP); KURIHARA Toshihide, Tokyo 160-0016 (JP); TSUBOTA Kazuo, Tokyo 160-0016 (JP)
(74) Representative: Mewburn Ellis LLP
(86) International application number: PCT/JP2021/034202
(87) International publication number: WO 2022/059757

(57) **Abstract**

To provide a device and a method for inhibiting thinning of a choroid.

The above-described problem is solved by a choroidal thinning inhibiting device comprising a light source that emits violet light having a wavelength within a range from 360 nm to 400 nm, the choroidal thinning inhibiting device inhibiting thinning of a choroid by irradiation with the violet light. This device may further comprise a control mechanism that controls an irradiance and an irradiation time of the violet light, can be applied to ocular diseases selected from age-related macular degeneration, glaucoma, diabetic retinopathy, macular edema, eye strain, retinal vascular occlusion, triangle syndrome, central serous chorioretinopathy, retinitis pigmentosa, presbyopia, cataracts, and the like that are based on inadequate blood flow or reduced blood flow, and can be expected to prevent and treat such ocular diseases.

## Description

### Field of the Invention

The present invention relates to a choroidal thinning inhibiting device and a choroidal thinning inhibiting method that inhibit thinning of a choroid to maintain a healthy eye by irradiation with violet light.

### BACKGROUND ART

The choroid is a membrane on an inner side of the sclera, which is the so-called white part of the eye. The choroid supplies oxygen and nutrients to the eyeball and retina through internal blood vessels. On the other hand, choroidal thinning is known to cause inadequate blood flow in the eye. About 80% of the blood flow in the eye is reportedly choroidal blood flow, which plays a significant role in maintaining eye health, improving eye function, advancing and improving ocular diseases, and the like (Non-Patent Document 1).

### PRIOR ART DOCUMENTS

### Non-Patent Documents

Non-Patent Document 1: Nickla, D.L. & Wallman, J. The multifunctional choroid. Prog Retin Eye Res 29, 144-168, Doi: 10. 1016 / j. preteyeres. 2009. 12. 002 (2010)
Non-Patent Document 2: Mori, K. et al. Scientific reports 9, 295, Doi: 10. 1038 / s41598-018-36576-w (2019)

### SUMMARY OF THE INVENTION

### Problems to be Solved by the Invention

An object of the present invention is to provide a device and a method for inhibiting thinning of a choroid.

### Means for Solving the Problems

(1) A choroidal thinning inhibiting device according to the present invention comprises a light source that emits violet light having a wavelength within a range from 360 nm to 400 nm, and inhibits thinning of a choroid by irradiation with the violet light.

The choroidal thinning inhibiting device according to the present invention further comprises a control mechanism that controls an irradiance and an irradiation time of the violet light.

In the choroidal thinning inhibiting device according to the present invention, applicable targets include ocular diseases selected from age-related macular degeneration, glaucoma, diabetic retinopathy, macular edema, eye strain, retinal vascular occlusion, triangle syndrome, central serous chorioretinopathy, retinitis pigmentosa, presbyopia, cataracts, and the like that are based on inadequate blood flow or reduced blood flow.

In the choroidal thinning inhibiting device according to the present invention, applicable targets include systemic symptoms and diseases selected from arteriosclerosis obliterans, thromboangiitis obliterans, diabetes, myocardial infarction, angina pectoris, stroke, cold limbs, pain, hair loss, tinnitus, stiff shoulders, swelling, cold constitution, menstrual irregularities, autonomic nervous disorder, chronic fatigue, sleepiness, constipation, and the like that are based on inadequate blood flow or reduced blood flow.

(2) A choroidal thinning inhibiting method according to the present invention comprises emitting violet light having a wavelength within a range from 360 nm to 400 nm to inhibit thinning of a choroid.

The choroidal thinning inhibiting method according to the present invention further comprises controlling an irradiance and an irradiation time of the violet light.

In the choroidal thinning inhibiting method according to the present invention, the applicable targets of the choroidal thinning inhibiting device according to the above-described present invention are similarly applied.

### Effect of the Invention

According to the present invention, it is possible to provide a device and a method for inhibiting thinning of a choroid. In particular, it is possible to inhibit the thinning of the choroid, through which most of the blood flow in the eye flows, and thus expect improvement and treatment of various symptoms and diseases that are based on inadequate blood flow or reduced blood flow.

### BRIEF DESCRIPTION OF THE DRAWINGS

Fig. 1 is a graph of results of Experiment 1 indicating thicknesses of choroids.
Fig. 2 is a photograph showing induction of scleral thinning inhibition in mice.
Fig. 3 is an explanatory view of measurements of scleral thickness taken at positions of 300 µm above (+ side) and below (- side) an optic disk, with a position of the optic disk set at "0." Fig. 4 is a graph of results of Experiment 2 indicating thicknesses of sclerae.

### Embodiments of the Invention

A choroidal thinning inhibiting device and a choroidal thinning inhibiting method according to the present invention will now be described. The present invention is not limited to the contents of the following embodiments and examples, and includes various modifications and applications within the scope of the gist of the present invention.

### [Choroidal Thinning Inhibiting Device and Choroidal Thinning Inhibiting Method]

The choroidal thinning inhibiting device and the choroidal thinning inhibiting method according to the present invention emit violet light having a wavelength within a range from 360 nm to 400 nm, and inhibit the thinning of the choroid by irradiation with the violet light. Such a device and a method, as shown in experiment results described below, make it possible to inhibit the thinning of the choroid through which most of the blood flow in the eye flows, adequately supply oxygen and nutrients to the eyeball and retina, and thus expect improvement and treatment of various symptoms and diseases that are based on inadequate blood flow or reduced blood flow.

The present invention at least inhibits the thinning of the choroid by irradiation with the violet light. "At least inhibits the thinning" includes not only inhibiting the thinning of the choroid but also thickening (increasing the thickness of) the choroid. Such a thinning inhibition or a thickness increase of the choroid maintains or increases the blood flow in the choroid, making it possible to use (emit) violet light to improve various symptoms and treat diseases that are based on inadequate blood flow or reduced blood flow. Such findings were previously completely unknown and were first discovered by the present inventors.

### (Violet light)

Violet light has a wavelength within a range from 360 to 400 nm. "Irradiation with violet light" refers to irradiation with light having a wavelength within a range from 360 to 400 nm, in whole or in part. Violet light is emitted from a light source. Such a light source may emit light having all wavelengths within the above-described wavelength range, may emit light having some (a specific range) of the wavelengths within the wavelength range, or may emit a spectrum having a peak wavelength within the wavelength range. It should be noted that, in the case of emitting light having some of the wavelengths, a light source that emits only some of the range may be used, or a light source that blocks light having a wide wavelength range by a filter and emits only light having some of the wavelengths may be used. Further, as long as violet light within the range from 360 to 400 nm is included, the light source may include light less than 360 nm or include light greater than 400 nm. Furthermore, in the vicinity of 360 nm and the vicinity of 400 nm, the light source may include a small amount of light less than 360 nm or may include a small amount of light greater than 400 nm is acceptable. In particular, as in an example described below, a violet light source having a maximum peak within the range from 360 to 400 nm is preferably applicable, and specifically a violet light source having a maximum peak within the range from 375 to 380 nm is preferred.

As long as within the wavelength range (from 360 to 400 nm) of violet light, the light source is not particularly limited. However, a light source having a peak at 380 nm or a light source having a peak within a range from 365 to 380 nm, for example, can be preferably used from the perspective of the availability of light sources on the market. It should be noted that the light source may emit light other than violet light together with the violet light, or may emit only violet light. Usually, a light source that emits only violet light is preferably adopted. The light other than violet light may be, for example, light greater than 400 nm or light less than 360 nm. However, light equal to or less than 315 nm has adverse effects on the eye, and thus is desirable to not include light less than 350 nm to the extent possible in view of safety.

An irradiance of the violet light is preferably within a range from 0.01 to 5 mW/cm². With this range of irradiance, it is possible to exhibit the effect of the present invention. More preferably, the irradiance is within a range from 0.01 to 1 mW/cm², which is a safer range for the eye and allows irradiation for a longer time. It should be noted that, in the experiments described below, irradiation was performed at an irradiance of 0.4 mW/cm² (400 µW/cm²), and could be performed within a preferred range from 0.1 to 0.8 mW/cm² around this irradiance.

The device according to the present invention and equipped with the above-described light source may be an installation-type irradiation device or illumination device, or may be a portable-type irradiation device or illumination device. In particular, because irradiation can be performed at low irradiance levels as described above, the device is preferably a portable-type irradiation device or illumination device and, as long as a device that is mounted onto the face, such as a face mask, for example, or a device such as eyeglasses, can be utilized in daily life. It should be noted that "irradiation device" is used to mean a device that emits at least violet light, and "illumination device" is used to mean a device that emits violet light together with white light.

An irradiation time of the violet light is set as desired in consideration of its effect. For example, in the example with mice described below, results were obtained in an experiment in which mice were irradiated for three hours. For humans, however, as long as there is no interference with normal daily life, the time is not particularly limited and examples include several hours per day (one to five hours, for example). A duration may range from a few weeks (two to eight weeks, for example) to a few months (two to six months, for example). By the irradiation with violet light over such a time and a duration, irradiation with weak light can be performed on a daily basis, comfortably in a living environment. It should be noted that the light can be made intermittent (regular intervals or irregular intervals) or continuous as desired, and thus the irradiation time and duration can be set in consideration of such irradiation modes.

### (Control mechanism)

The control mechanism is a mechanism that controls the emission of light from the light source, and executes control so that a surface of the eye can be irradiated with violet light at an irradiance within the above-described range (from 0.01 to 5 mW/cm²). This control mechanism may be a mechanism capable of setting the irradiation time in accordance with the irradiance. Specifically, examples include a control mechanism that controls the irradiance and an irradiation method (continuously, at regular intervals, or the like) or controls the irradiation time per day and the duration (daily, every other day, or the like, for example). The control mechanism may be an integral part of the light source or may be a control device separate from the light source. An "integral part" refers to, for example, a case in which a light-emitting diode (LED) and a control circuit are integrated to form the light source, and a "separate device" refers to, for example, a case in which a light source composed of an LED and a control device that controls the emission of the LED in a wired or wireless manner are configured as separate members.

The control mechanism preferably includes a timer device (including a clock), a storage device (memory), a display device (display panel), a communication device, and the like. With these devices, it is possible to control the irradiation time per day, store information in a case of repeated irradiation, display such information on a liquid crystal display panel, transmit and receive data to and from a smartphone or personal computer for control and data accumulation by application software, and the like, for example. Further, if the control mechanism is equipped with a transmission device that transmits data to hospitals and physicians, management by hospitals and physicians is possible even at remote locations, enabling appropriate management and treatment guidance.

### (Inhibition of choroidal thinning)

The choroidal thinning inhibiting device and the choroidal thinning inhibiting method according to the present invention and having such a configuration at least inhibit the thinning of the choroid. "At least" means that the irradiation with violet light at least inhibits the thinning of the choroid. Accordingly, in addition to maintaining a thickness (inhibiting the thinning) of the choroid by irradiation with violet light, the meaning may include a case in which the choroid is thickened. With the inhibition of the thinning of the choroid, it is possible to maintain the current state of blood flow in the choroid. Further, with the thickening of the choroid, it is possible to improve the current state of blood flow in the choroid and increase the blood flow. Improving the blood flow can improve the state of health of the eye, can be expected to improve or treat ocular diseases caused by blood flow conditions in the eye, and can be expected to improve or treat systemic symptoms and diseases caused by blood flow conditions in the eye.

That is, a choroidal thinning inhibiting means confirmed in the experiments can be expected to have progression preventive effects and therapeutic effects on other ocular diseases associated with choroidal thinning, specifically, ocular diseases, systemic symptoms, and diseases in which the choroidal thinning is reportedly based on inadequate blood flow or reduced blood flow in the eye. Examples of ocular diseases based on inadequate blood flow or reduced blood flow include age-related macular degeneration, glaucoma, diabetic retinopathy, macular edema, eye strain, retinal vascular occlusion, triangle syndrome, central serous chorioretinopathy, retinitis pigmentosa, presbyopia, cataracts, and the like. Further, examples of systemic symptoms and diseases caused by inadequate blood flow or reduced blood flow include arteriosclerosis obliterans, thromboangiitis obliterans, diabetes, myocardial infarction, angina pectoris, stroke, cold limbs, pain, hair loss, tinnitus, stiff shoulders, swelling, cold constitution, menstrual irregularities, autonomic nervous disorder, chronic fatigue, sleepiness, constipation, and the like.

### (Inhibition of scleral thinning)

The choroidal thinning inhibiting device and the choroidal thinning inhibiting method according to the present invention at least inhibit the thinning of the choroid and, as understood from the results of Experiment 2 described below, can inhibit the thinning of the sclera as well. Inhibiting the thinning of the sclera means at least inhibiting the thinning of the sclera by irradiation with violet light. Accordingly, in addition to maintaining a thickness (inhibiting the thinning) of the sclera by irradiation with violet light, the meaning may include a case in which the sclera is thickened. Inhibiting the thinning of the sclera has the effect of inhibiting elongation of an ocular axial length and maintaining a shape of the eyeball. In the present invention, however, irradiation with violet light can simultaneously inhibit the thinning of the choroid and inhibit the thinning of the sclera. Thus, by inhibiting the thinning of the choroid and improving blood flow, it is possible to achieve a synergistic effect of increasing (maintaining) the supply of oxygen and nutrients to the sclera, appropriately remodeling an extracellular matrix of the sclera, inhibiting scleral thinning, and maintaining the shape of the eyeball.

### Examples

The present invention will be described below in further detail using experimental examples.

### [Experiment 1]

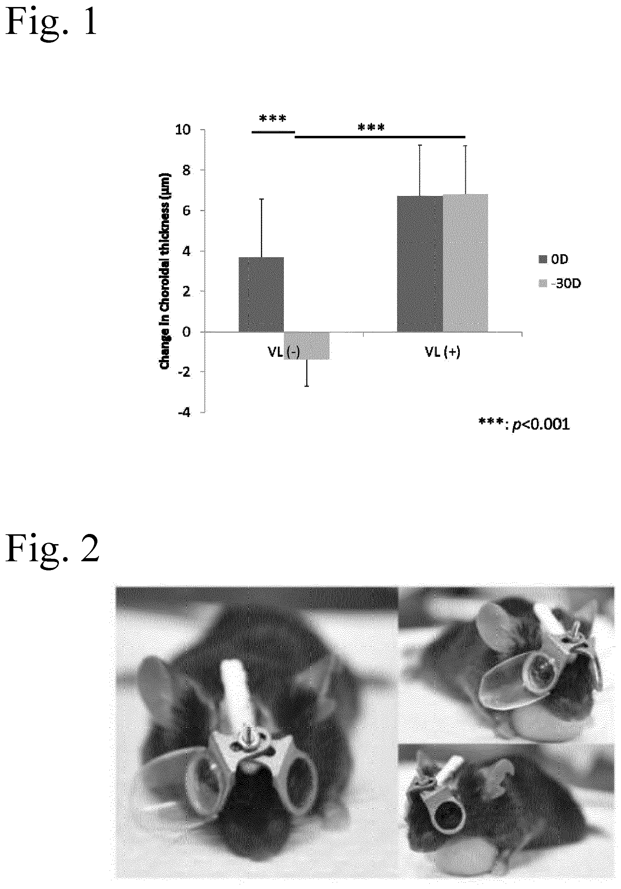

A 0D ("D" is an abbreviation for diopter and is a unit of refractive power of a lens) lens and a -30D lens (Rainbow Contact, Rainbow Optical Laboratory Co., Ltd.) were mounted onto left eyes and right eyes, respectively, of three-week-old mice (C57BL6/J, Clea Japan, Inc.) (refer to Fig. 2) to induce inhibition of choroidal thinning. For lens attachment, a support column was erected on the cranium and fixed with dental cement (Super-Bond, Sun Medical Co., Ltd.). The support column was provided with threads so that an adjustment instrument described below could be secured with a nut. The inhibition of choroidal thinning was initially induced after randomly dividing the mice into VL (+) and VL (-) groups, with five mice in each group. "VL" herein is an abbreviation for violet light. The VL (-) group was irradiated with a 5000-Kelvin fluorescent light of around 50 Lux as background light from 8 a.m. to 8 p.m. daily, and the VL (+) group was irradiated with a violet light (LED light source: Nichia Corporation, model number: NSPU510CS, peak wavelength: 375 nm) of 400 µW/cm² at wavelengths from 360 to 400 nm, from 5 p.m. to 8 p.m. daily, in addition to the above background light. The irradiation was continued for three weeks, and a refractive index, an ocular axial length, and a choroidal thickness of the mouse eyeball before and after irradiation were measured and the amounts of change were calculated.

The above-described light source used had a peak wavelength at 375 nm and, given 1 (100%) as the spectral irradiance at that peak wavelength, a relative spectral irradiance of less than 0.025 (2.5%) at 360 nm and a relative spectral irradiance of less than 0.025 (2.5%) at 400 nm as well.

### (Method of choroidal measurement)

The choroid varies in thickness from place to place, and thus an SD-OCT (Envisu R4310, Leica, Germany) was used to measure the choroid of each mouse at the optic nerve center in a circle having a 0.5 mm radius. The area of the choroid was measured with Image J (National Institutes of Health, Bethesda, USA) (using the same method as in Non-Patent Document 2), and the average choroidal thickness was obtained by dividing the value by the ratio of the circumference of a circle to its diameter.

The measurement results are shown in Fig. 1. The vertical axis indicates choroidal thickness, and the results of 0D in the left eye and -30D in the right eye in the VL (-) and VL (+) groups are compared. The results of Fig. 1 shows that the mice in the VL (+) group that were additionally irradiated with violet light increased in choroidal thickness after irradiation in both the right and left eyes compared to the mice in the VL (-) group that were not additionally irradiated with violet light. Especially in -30D in the right eye, the mice in the VL (-) group without additional violet light irradiation exhibited the thinning of the choroid, while the mice in the VL (+) group with additional violet light irradiation increased in the thickness of the choroid, and the difference was extremely remarkable. This result means that violet light irradiation acts to increase the thickness of the choroid and improve choroidal blood flow. 80% of the blood flow in the eye is choroidal blood flow, and therefore the thickening of the choroid makes it possible to improve and increase the blood flow in the eye and, as a result, expect to improve and treat ocular diseases caused by blood flow conditions in the eye, and expect to improve and treat systemic symptoms and diseases caused by blood flow conditions in the eye.

### [Experiment 2]

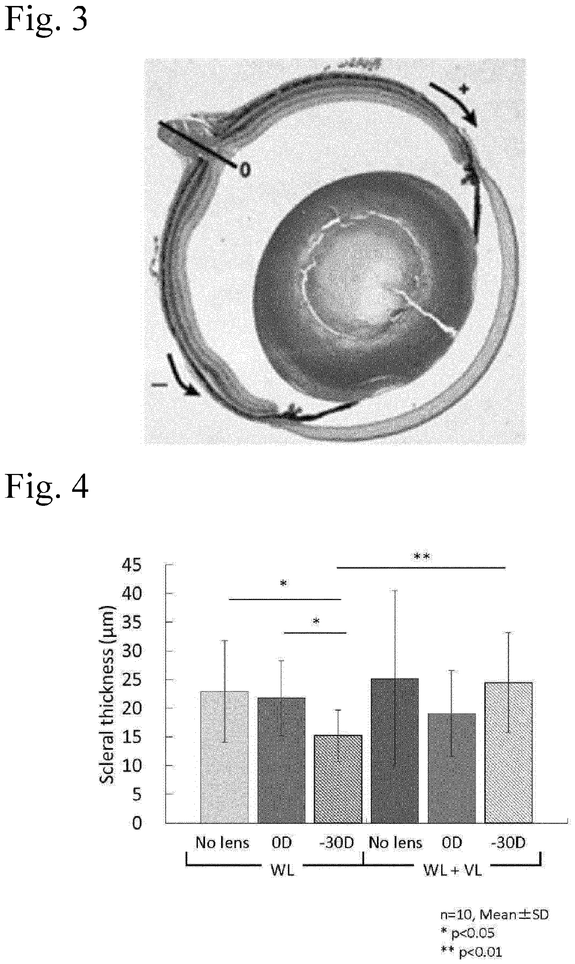

As in Experiment 1, three-week-old mice (C57BL6/J, Clea Japan, Inc.) were used. The mice were randomly divided into (i) a no lens group, a 0D group ("D" is an abbreviation for diopter and is a unit of refractive power of a lens; a group mounted with the same 0D lenses as in Experiment 1 on both eyes as shown in Fig. 2), and a -30D group (a group mounted with the same -30D lenses as in Experiment 1 on both eyes as shown in Fig. 2) irradiated with white light (WL), and into (ii) a no lens group, a 0D group (the same as described above), and a -30D group (the same as described above) irradiated with violet light (VL), with five mice in each group. The mounting of the lenses was the same as in Experiment 1. "Irradiated with white light" herein is a mode in which the groups were irradiated with a 5000-Kelvin fluorescent light of around 50 Lux as background light from 8:00 a.m. to 8:00 p.m. daily. "Irradiated with violet light" is a mode in which the groups were irradiated with violet light (LED light source: Nichia Corporation, model number: NSPU510CS, peak wavelength: 375 nm) of 400 µW/cm² at wavelengths from 360 to 400 nm, from 5 p.m. to 8 p.m. daily, in addition to the above background light. Both irradiations were continued for three weeks, the sclerae of the mice were measured after irradiation, and the results are shown in Fig. 4.

It should be noted that the above-described light source used had a peak wavelength at 375 nm and, given 1 (100%) as the spectral irradiance at that peak wavelength, a relative spectral irradiance of less than 0.025 (2.5%) at 360 nm and a relative spectral irradiance of less than 0.025 (2.5%) at 400 nm as well.

### (Method of scleral measurement)

After the experiment, the eyeballs were extracted from the C57BL6J mice. The extracted eyeballs were fixed overnight in a 4% paraformaldehyde-phosphate buffer solution and sliced by a microtome (REM-710, Yamato Kohki Industrial Co., Ltd., Saitama, Japan) into horizontally cut paraffin sections (thickness: approximately 3 µm) at a position where the optic disk was visible. The sections were then stained with hematoxylin-eosin and made visible using a BX53 microscope (Olympus Corporation, Tokyo, Japan). The scleral thicknesses of the five mice in each group were measured using cellSens software (Olympus Corporation) at two points (above (+) and below (-)) 300 µm from the optic disk, as shown in Fig. 2.

The measurement results are shown in Fig. 4. The sclerae of the eyes irradiated with VL (irradiated with WL and VL, mounted with -30D) were thicker than those of the eyes in which scleral thinning inhibition was induced (irradiated with WL, mounted with -30D), indicating a scleral thinning inhibition effect. Thinning of the sclera increases elongation of the ocular axial length. However, with the thinning of the sclera inhibited, it is possible to inhibit elongation of the ocular axial length and maintain the shape of the eyeball, as in the results of Experiment 2.

The results of Experiment 1 and Experiment 2 show that VL irradiation can, in addition to inhibiting the thinning (including increasing the thickness) of the choroid in Experiment 1, inhibit the thinning of the sclera. Thus, in addition to the choroidal thinning inhibition effect (blood flow maintenance and blood flow improvement), the scleral thinning inhibition effect described above can be achieved simultaneously. Choroidal thinning is thought to cause scleral thinning, resulting in failure to maintain the shape of the eyeball and elongation of the ocular axial length. However, based on the results of Experiment 1 and Experiment 2, the thinning of the choroid and sclera can be simultaneously inhibited and thus, by inhibiting the thinning of the choroid and improving blood flow, it is possible to increase (maintain) the supply of oxygen and nutrients to the sclera, appropriately remodel the extracellular matrix of the sclera, inhibit scleral thinning, and maintain the shape of the eyeball.

## Claims

1. A choroidal thinning inhibiting device comprising:
a light source that emits violet light having a wavelength within a range from 360 nm to 400 nm,
the choroidal thinning inhibiting device inhibiting thinning of a choroid by irradiation with the violet light.

2. The choroidal thinning inhibiting device according to claim 1, further comprising a control mechanism that controls an irradiance and an irradiation time of the violet light.

3. The choroidal thinning inhibiting device according to claim 1 or 2, wherein
applicable targets include ocular diseases selected from age-related macular degeneration, glaucoma, diabetic retinopathy, macular edema, eye strain, retinal vascular occlusion, triangle syndrome, central serous chorioretinopathy, retinitis pigmentosa, presbyopia, cataracts, and the like that are based on inadequate blood flow or reduced blood flow.

4. The choroidal thinning inhibiting device according to claim 1 or 2, wherein
applicable targets include systemic symptoms and diseases selected from arteriosclerosis obliterans, thromboangiitis obliterans, diabetes, myocardial infarction, angina pectoris, stroke, cold limbs, pain, hair loss, tinnitus, stiff shoulders, swelling, cold constitution, menstrual irregularities, autonomic nervous disorder, chronic fatigue, sleepiness, constipation, and the like that are based on inadequate blood flow or reduced blood flow.

5. The choroidal thinning inhibiting device according to claim 1 or 2, wherein
thinning of a sclera is simultaneously inhibited.

6. A choroidal thinning inhibiting method comprising:
emitting violet light having a wavelength within a range from 360 nm to 400 nm to inhibit thinning of a choroid of an eye.

7. The choroidal thinning inhibiting method according to claim 6, further comprising controlling an irradiance and an irradiation time of the violet light.

8. The choroidal thinning inhibiting method according to claim 6 or 7, wherein
the applicable targets of the choroidal thinning inhibiting device described in any one of claims 3 to 5 are similarly applied.
